# EUROPEAN PATENT SPECIFICATION

(11) **EP 0 606 296 B1**
(45) Date of publication and mention of the grant of the patent: **03.03.1999**
(21) Application number: 92920269.5
(22) Date of filing: 18.09.1992
(51) Int. Cl.: C12Q 1/00, C12Q 1/54, C12Q 1/60

(54) **REAGENTS AND ASSAY METHODS INCLUDING A PHENAZINE-CONTAINING INDICATOR**
REAGENZIEN UND UNTERSUCHUNGSVERFAHREN EINSCHLIESSLICH EINES PHENAZIN ENTHALTENDEN INDIKATORS
REACTIFS ET METHODES DE DOSAGE COMPORTANT UN INDICATEUR CONTENANT DE LA PHENAZINE

(30) Priority: 19.09.1991 US 762278
(43) Date of publication of application: 20.07.1994
(73) Proprietor: BOEHRINGER MANNHEIM CORPORATION, Indianapolis, Indiana 46250-0528 (US)
(72) Inventor: WILSEY, Christopher, D., Carmel, IN 46032 (US); FREITAG, Helmut, D-6940 Weinheim (US)
(74) Representative: Jung, Michael, Dr.
(86) International application number: US9208039
(87) International publication number: WO9306487

(56) References cited:
- US-A- 4 243 539
- US-A- 4 391 904
- US-A- 4 472 498
- US-A- 4 803 161
- US-A- 4 849 330
- US-A- 4 853 186
- US-A- 4 857 271
- US-A- 4 912 035
- US-A- 5 013 669

## Description

### FIELD OF THE INVENTION

This invention relates to the colorimetric measurement of the amount of an analyte in a sample.

### BACKGROUND OF THE INVENTION

In colorimetric assays for measuring the amount of an analyte in a sample, phenazine-containing compounds have been used as mediators (redox mediators) in oxidation-reduction reactions to facilitate the reduction of an indicator. In such assays, the color intensity of the reduced form of the indicator is correlated to the amount of analyte in the sample. The following reaction sequences are exemplary of these assays:

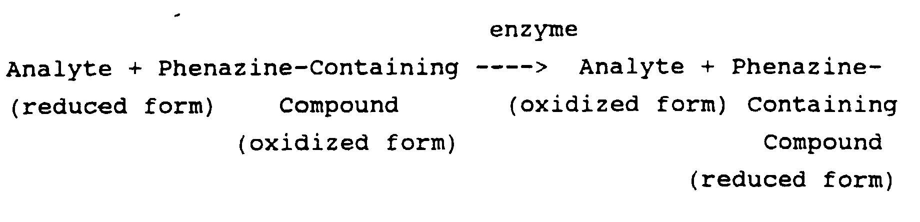

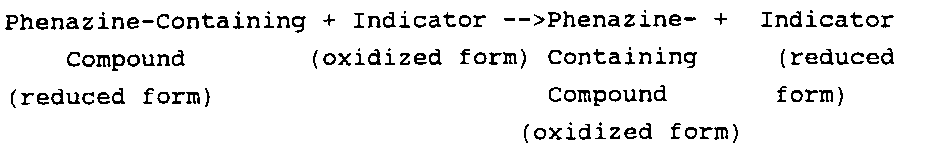

Phenazine-Containing Compound (oxidized form)= e.g., PMS (phenazine methosulfate)

Indicator (oxidized form) = e.g., tetrazolium salt, NAD (oxidized form of nicotinamide adenine dinucleotide)

Indicator (reduced form) = e.g., formazan, NADH (reduced form of nicotinamide adenine dinucleotide)

When used as redox mediators, phenazine-containing compounds function as non-enzymatic catalysts and their concentration in an assay is very low (significantly less than one millimolar).

### SUMMARY OF THE INVENTION

The invention is a new reagent and methods for measuring the concentration of (or detecting the presence of) an analyte in a sample. The reagent includes a phenazine-containing compound and an enzyme. The phenazine-containing compound must be of sufficient type to form a semiquinoid (the color indicator) by reaction involving the enzyme, analyte, and phenazine-containing compound. Importantly, the phenazine-containing compound must be in sufficient amount to correlate the concentration of semiquinoid to the concentration of analyte in the sample (or to detect the presence of the analyte in the sample).

The reagent may also include the following components: A buffer to provide and maintain a proper pH for the reaction involving, enzyme, analyte, and phenazine-containing compound and to provide a desired pH for spectrophotometric measurements of the semiquinoid; and a surfactant in sufficient amount to prevent precipitation of the semiquinoid.

A reagent kit for measuring the amount of an analyte in a sample may also be provided. In the reagent kit, a first reagent includes the phenazine-containing compound, and a second reagent includes the enzyme and the buffer.

The inventive reagent may be incorporated into a film, which at a minimum includes the phenazine-containing compound, enzyme, and a film forming agent, such as NATROSOL-250M, which is a micro-crystalline hydroxyethylcellulose, available from Aqualon Company (Little Falls Centre One, 2711 Centerville Road, P.O. Box 15417, Wilmington, Delaware 19850-5417). The film may further include a buffer, a reagent stabilizer, and a surfactant.

Alternatively, the inventive reagent may impregnate a fabric mesh (such as a nylon mesh) or paper. The reagent may also be coated on glass fibers.

The methods for measuring the amount of (or detecting the presence of) an analyte in a sample importantly include spectrophotometric measurement (or detection) of the semiquinoid indicator at wavelengths greater than about 580 nanometers, which reduces interferences due to the presence of hemoglobin, bilirubin, and turbidity. Further, these assay methods importantly involve short incubation periods of less than about 30 seconds for test samples measuring spectrophotometric absorbance (assays performed on solutions) and less than about 1.5 minutes for test samples measuring spectrophotometric reflectance or transmittance (assays performed on films).

### DETAILED DESCRIPTION OF THE INVENTION

Traditionally, phenazine-containing compounds have been used in low concentration as redox mediators (electron carriers) in the assay of analytes. For example, a phenazine-containing compound typically acts as a redox mediator in a reaction involving an enzyme, an analyte, and a dye, such as a tetrazolium salt. In such a reaction, the phenazine-containing compound acts as a redox mediator in reducing the dye. The reduced dye is a color indicator, such as a formazan, which is used in measuring the amount of analyte in a sample.

In US-A 4,853,186 phenazine-containing compounds are disclosed which act as electron transfer agents (ETA). These ETA transfer electrons to reducible compounds which in turn release a detectable species, e.g. a dye, in assays for e.g. microorganisms.

US-A 4,472,498 discloses the use of phenazine-containing compounds in analysis films. As in US-A 4,853,186 the phenazine-containing compounds of US-A 4,472,498 are used to reversibly form a semiquinoid whereby the actual detection reaction is achieved by further means, e.g. a further enzyme-substrate couple.

However, if a phenazine-containing compound is supplied in high enough concentration relative to the amount of analyte being measured, it behaves as an indicator rather than merely as an electron carrier in the assay of an analyte.

When spectrophotometric measurements of a solution are performed, the present inventive reagent minimally includes a phenazine-containing compound and an enzyme.

However, N-ethylmethoxyphenazine ethosulfate and 1-methoxyphenazine methosulfate, as well as many other phenazine-containing compounds, are red colored, which creates a high blank reaction in an assay of an analyte. Therefore, phenazine ethosulfate and phenazine methosulfate are preferred phenazine-containing compounds because they are yellow colored and do not create a high blank reaction in the assay of an analyte.

To correlate the concentration of semiquinoid to the concentration of glucose in a blood sample, the concentration of phenazine-containing compound in a reagent should be at least about four (4) millimolar (mM). For detection, rather than measurement, of glucose in a blood sample, the concentration of phenazine-containing compound in a reagent may be as low as about one (1) mM.

The minimum amount of phenazine-containing compound required in the reagent to measure or detect a particular analyte in a sample will depend upon the following factors:
1) the concentration of the analyte being measured;
2) the efficiency of the enzyme as a redox catalyst;
3) the numbers of transferred electrons;
4) if the enzyme is an oxidase, the efficiency of the oxidase's reaction with oxygen, which competes with phenazine-containing compound for reaction and the amount of oxygen available to react with the oxidase (that is, the more efficient the oxidase's reaction with oxygen and the more oxygen available to react with the oxidase, the more phenazine-containing compound needed in the reagent). The upper limit of the amount of phenazine-containing compound that may be provided in the reagent is limited by the solubility of the compound in the reagent.

The enzyme supplied to the reagent must be of sufficient type and in sufficient amount to catalyze the reaction involving enzyme, analyte, and phenazine-containing compound. For example, if glucose is the analyte sought to be measured, the enzyme may be glucose oxidase. Likewise, cholesterol oxidase may be used in analyzing cholesterol and glycerol-3-phosphate oxidase may be used in analyzing glycerol-3-phosphate.

A buffer may sometimes be a required or a preferred additive to the reagent. The buffer must be of sufficient type and in sufficient amount to provide and maintain a proper pH for the reaction involving enzyme, analyte, and phenazine-containing compound. Examples of buffers that may be used in the reagent, depending upon the pH desired, include "Good" buffers, such as 2-(N-morpholino)ethanesulfonic acid, N-(2-acetamido) -2-iminodiacetic acid, piperazine- N,N'-bis (2-ethanesulfonic acid), N-(2-acetamido)-2-aminoethanesulfonic acid, N,N-bis (2-hydroxyethyl)-2-aminoethanesulfonic acid, N-tris (hydroxymethyl)methyl-2-aminoethanesulfonic acid, and N-2-hydroxyethylpiperazine - N'-2- ethanesulfonic acid. Maleic acid may also be used for glucose analysis.

A surfactant may also preferably be added to the reagent, particularly at higher phenazine-containing compound concentrations. The surfactant must be of sufficient type and in sufficient amount to prevent precipitation of the semiquinoid indicator. Nonionic surfactants, such as polyoxyethylene ethers, polyoxyethylene sorbitans, and TRITON surfactants (available from Sigma Chemical Company) may be used.

Specific reagents for the analysis of specific analytes may be formulated as follows:

### Glucose Reagent

Step 1 - A buffer stock solution was prepared by dissolving about 3.9 grams (g) of 2-(N-morpholino) ethanesulfonic acid in about 100 milliliters (ml) of distilled water. (The concentration of 2-(N-morpholino) ethanesulfonic acid in the buffer stock solution was about 200mM.) The pH of the resulting solution was adjusted to about 5.6 with sodium hydroxide.

Step 2 - An enzyme-containing solution was prepared by dissolving about 6 kilounits (ku) of glucose oxidase (Aspergillus niger) in about 8 ml of the buffer stock solution.

Step 3 - A phenazine-containing compound solution was prepared by dissolving about 3.3 g of phenazine ethosulfate in about 25 ml of distilled water. (This solution was about 400 mM in phenazine ethosulfate.)

A single glucose reagent may be prepared by combining the enzyme-containing solution with the phenazine-containing compound solution in a ratio of about 9:1 (volume:volume). Alternatively, a reagent kit may be provided, wherein the enzyme-containing solution and the phenazine-containing compound solution are each kept in separate vials (and may be lyophilized). When using the reagent kit, the enzyme-containing solution and the phenazine-containing compound solution should be combined in the above stated ratio (about 9:1) (volume:volume) to perform a glucose assay. (Lyophilized vials may also be reconstituted with water and combined in the above-stated ratio.)

Step 1 - A buffer stock solution may be prepared by dissolving about 6.3 g of 3-(N-morpholino) propanesulfonic acid in about 100 ml of distilled water. (The concentration of 3-(N-morpholino) propanesulfonic acid in the buffer stock solution will be about 300 mM.) The pH of the buffer stock solution may be adjusted to about 7.2 by the addition of 1 Normal (N) sodium hydroxide.

Step 2 - About 2.2 ku of cholesterol oxidase (from Nocardia erythropolis) and about 104 milligrams (mg) of phenazine ethosulfate may be dissolved in 5.2 ml of the buffer stock solution. To the resulting solution, TRITON X-100 (a nonionic surfactant, available from Sigma Chemical Company) may be added in an amount which makes the resulting solution (the cholesterol reagent) one percent (volume:volume) TRITON X-100.

The cholesterol reagent may be provided as a single reagent or as a reagent kit. In a reagent kit, phenazine ethosulfate (preferably in lyophilized form) is provided in one container, and in a separate container the other reagent ingredients (preferably lyophilized) are provided.

### Glycerol-3-Phosphate Reagent

Step 1 - A buffer stock solution was prepared by dissolving about 3.6 g of N-2-hydroxyethylpiperazine-N/-2-ethanesulfonic acid in about 100 ml of distilled water. The pH of the resulting solution was adjusted to about 7.6 by the addition of 1N sodium hydroxide.

Step 2 - An enzyme-containing solution was prepared by dissolving about 8 ku of glycerol-3-phosphate oxidase in about 8 ml of buffer stock solution.

Step 3 - A phenazine-containing compound solution was prepared by dissolving about 3.3 g of phenazine ethosulfate in about 25 ml of distilled water. (The resulting concentration of phenazine ethosulfate was about 400 mM.

A single glycerol-3-phosphate reagent may be obtained by combining the enzyme-containing solution with the phenazine-containing compound solution at a ratio of about 9:1 (volume:volume). A glycerol-3-phosphate reagent kit may also be provided by keeping the enzyme-containing solution and the phenazine-containing compound solution separate. (Each of these solutions is preferably lyophilized.) When the kit is used for an assay of glycerol-3-phosphate, the enzyme-containing reagent and the phenazine-containing compound reagent should be provided in the same proportions as specified above for the single glycerol-3-phosphate reagent. (These proportions also apply to kit reagents that are lyophilized and subsequently reconstituted by the addition of water.)

The above stated reagents are liquid reagents (or are liquid after lyophilized reagent is reconstituted with water). These reagents may also be incorporated into films. When incorporated into a film, the reagent minimally includes a phenazine-containing compound, an enzyme, and a film forming agent, such as a microcrystalline hydroxyethylcellulose.

As stated above for liquid and lyophilized reagents, in a film the phenazine-containing compound must be of sufficient type to form a semiquinoid by the reaction involving enzyme, analyte, and phenazine-containing compound and must be in sufficient amount to detect the semiquinoid (thereby detecting the presence of the analyte) or to correlate the concentration of semiquinoid to the concentration of analyte in the sample being analyzed. Accordingly, the amount of phenazine-containing compound incorporated into the film for glucose analysis of a blood sample should be at least about 18 micromoles (µ mol) per g of dry film (assuming 100% dry or 100% solids in the film) for correlating the concentration of semiquinoid to the concentration of glucose in the sample being analyzed and at least about 11 µ mol per g of dry film to detect the presence of the glucose in the sample. The enzyme must also be of sufficient type and in sufficient amount to catalyze the reaction involving enzyme, glucose, and phenazine-containing compound.

A buffer may also be incorporated into the film. The types of buffers that may be used and the requirements of those buffers are the same as those stated above for liquid and lyophilized reagents. (When films are used to perform assays, spectrophotometric reflectance or transmittance measurements are made rather than absorbance measurements.)

A reagent incorporated into a film may also include a surfactant. The surfactant must be of sufficient type and in sufficient amount to wet the surface of the film upon addition of the sample being analyzed.

A specific example of a film that may be used for glucose analysis is as follows:

| **Glucose Film** | |
|---|---|
| Film Component | Amount per kilogram of film (wet weight) |
| Malic Acid | 150 mM |
| Nickel Sulfate | 50 mM |
| Manganese Sulfate | 50 mM |
| ^{*a*} CELABRITE | 22% (weight:weight) |
| NATROSOL-250M | 0.75% (weight:weight) |
| Dextran Sulfate (molecular weight=5000g/mol) | 2% (weight:weight) |
| Glucose Oxidase (from Aspergillus Niger, from Biozyme Laboratories Limited) | 1500 units/g wet film available |
| ^{*b*} PROPIOFAN 70D | 7% (weight:weight) |
| ^{*c*} TWEEN 20 | 0.5% (weight:weight) |
| Phenazine Ethosulfate Water | 60 mM |

| | |
|---|---|
| ^{*a*}A diatomaceous earth, available from Eagle-PicherIndustries, Inc., Cincinnati, Ohio. | |
| ^{*b*}An aqueous vinyl propionate copolymer dispersion of large particle size, available from BASF Corporation. This composition contains a protective colloid. | |
| ^{*c*}Polyoxyethylenesorbitan monolaurate, available from Sigma Chemical Company. | |

This film may be coated onto 250 micrometer CRONAR plastic (a plastic with a gel backing available from DuPont). The wet coating may be dried at about 50° C for 20 minutes to remove more than 90% of the water in the wet film.

The present inventive reagent may be advantageously incorporated into methods for measuring the amount of an analyte in a sample. The general method for measuring the amount of (or alternatively detecting the presence of) an analyte in a sample includes the following steps:
Step 1 - forming a test sample by combining the sample containing the analyte with a single liquid reagent or a film (described above);
Step 2 - incubating the test samples;
Step 3 - spectrophotometrically measuring absorbance of the incubated test sample at a wavelength from about 520 nanometers (nm) to about 740 nm; and
Step 4 - correlating the measured absorbance of the incubated test sample to the amount (or to the presence) of analyte in the sample.

Importantly, the amount (or presence) of indicator (semiquinoid) may be spectrophotometrically measured (or detected) at wavelengths from about 580 to about 740 nm. Spectrophotometric measurement at these longer wavelengths decreases interference due to hemoglobin, bilirubin, and turbidity, which may be present in the sample being analyzed. Although spectrophotometric measurements may be made at wavelengths from about 520 to about 740 nm, spectrophotometric measurements are more preferably made at wavelengths from about 590 to about 710 nm and most preferably from about 620 to about 670 nm.

Another advantage of these methods for measuring an analyte in a sample is that the test sample incubation periods are much shorter than the test sample incubation periods of more traditional colorimetric assay methods. When a liquid reagent is used to measure the spectrophotometric absorbance of a solution, the incubation period may range from about 10 seconds to about 1 minute and will usually range from about 10 to about 40 seconds. When a film is used in the spectrophotometric measurements of reflectance or transmittance, the incubation period may range from about 20 seconds to about 1.5 minutes and will usually range from about 20 to about 60 seconds.

The present methods may be illustrated by the following examples:

### Example 1 - Glucose Assay

Aqueous (distilled water) glucose stock solutions at concentrations of 189, 472.5, 787.5, and 1,417.5 mg per deciliter (dl), respectively, were prepared. An assay was conducted by separately combining 50 microliters (µl) of each glucose stock solution with 1 ml of the single specifically formulated liquid glucose reagent described above, thereby forming a test sample. Each test sample was incubated for about 15 seconds at ambient temperature. Spectrophotometric absorbance of each incubated test sample was then measured at 646 nm. There was direct correlation between the spectrophotometric absorbance of the test sample and the amount of glucose in the sample (stock solution) being analyzed.

### Example 2 - Cholesterol Assay

PRECISET cholesterol standards (available from Boehringer Mannheim Corporation) may be used. Cholesterol standards of 0, 50, 100, 150, 200, 300, and 400 mg cholesterol per dl of standard, respectively, may be prepared. Assays of each cholesterol standard may be performed by combining 500 µl of cholesterol standard with 500 µl of the single specifically formulated liquid cholesterol reagent (described above), thereby forming a test sample. The test may be incubated for about 15 seconds at ambient temperature. Spectrophotometric absorbance of the test sample may then be measured at 602 nm. The intensity of spectrophotometric absorbance may be directly correlated to the amount of cholesterol in the cholesterol standard being analyzed.

### Example 3 - Glycerol-3-Phosphate Assay

Aqueous (distilled water) glycerol-3-phosphate standards of 22.1, 44.2, and 66.3 mM were prepared. Test samples were prepared by separately adding 50 µl of each glycerol-3-phosphate stock solution to 1 ml of the specifically formulated glycerol-3-phosphate liquid reagent described above. Each test sample was incubated for about 15 seconds at ambient temperature. The spectrophotometric absorbance of each incubated test sample was measured at 602 nm. The intensity of spectrophotometric absorbance directly correlated to the amount of glycerol-3-phosphate in each glycerol-3-phosphate standard.

When an assay method employs a reagent kit (as described above), the first and second reagent of the kit may be combined (to form a single liquid reagent, as described above) prior to the addition of the sample to be analyzed. Alternatively, an intermediate sample may be formed by combining the sample containing the analyte with the first reagent of the kit. (The first reagent includes the phenazine-containing compound.) A test sample is then formed by combining the intermediate sample with the second reagent of the kit. (The second reagent of the kit includes the enzyme and buffer.) The test sample is then incubated (as described above), and the incubated test sample is spectrophotometrically measured (as described above). In an assay, the incubation period should be triggered by addition of the enzyme or the analyte sought to be measured rather than the phenazine-containing compound. (If the analyte sought to be measured and the enzyme are combined prior to the addition of the phenazine-containing compound, an unwanted reaction involving enzyme, analyte, and oxygen may occur.)

When a film is used instead of a liquid reagent, the test sample is formed by combining a liquid sample containing the analyte with the film. (See Step 1 of the general method.) The test sample is then incubated from about 20 to about 60 seconds at ambient temperature. (See Step 2 of the general method.) Reflectance or transmittance of the incubated test sample is then measured (or detected) at the wavelengths specified above. (See Step 3 of the general method.) The intensity of reflectance or transmittance of the incubated test sample is inversely proportional to the amount of analyte in the sample being analyzed. (See Step 4 of the general method.)

The present invention has been disclosed in the above teachings with sufficient clarity and conciseness to enable one skilled in the art to make and use the invention, to know the best mode for carrying out the invention, and to distinguish it from other inventions and from what is old. Many variations and obvious adaptations will readily come to mind, and these are intended to be contained within the scope of the invention as claimed below.

## Claims

1. A reagent for detecting the presence of an analyte in a sample, comprising:
a phenazine-containing compound being of sufficient type to form a semiquinoid as indicator by a reaction involving enzyme, analyte, and the phenazine-containing compound and being in sufficient amount to allow detection of the semiquinoid indicator and thereby detection of the analyte in the sample, and
the enzyme being of sufficient type and in sufficient amount to catalyze the indicator-forming reaction involving enzyme, analyte, and phenazine-containing compound.

2. The reagent of claim 1, further comprising water.

3. The reagent of claim 1, wherein the phenazine-containing compound is in sufficient amount to correlate the concentration of semiquinoid to the concentration of analyte in the sample.

4. The reagent of claim 3, further comprising water.

5. The reagent of claim 2, wherein the analyte is glucose and the concentration of the phenazine-containing compound is at least about one millimolar.

6. The reagent of claim 4, wherein the analyte is glucose and the concentration of the phenazine-containing compound is at least about 4 millimolar.

7. The reagent of claim 4, wherein the phenazine-containing compound is selected from a group consisting of phenazine methosulfate, phenazine ethosulfate, N-ethylmethoxyphenazine ethosulfate, and 1-methoxyphenazine methosulfate.

8. The reagent of claim 4, wherein the phenazine-containing compound is selected from a group consisting of phenazine methosulfate and phenazine ethosulfate.

9. The reagent of any of claims 1 to 4, further comprising: a buffer of sufficient type and in sufficient amount to provide and maintain a proper pH for the reaction involving enzyme, analyte, and phenazine-containing compound.

10. The reagent of claim 8, further comprising:
a buffer of sufficient type and in sufficient amount to provide and maintain a proper pH for the reaction involving enzyme, analyte, and phenazine-containing compound.

11. The reagent of claim 9, further comprising:
a surfactant of sufficient type and in sufficient amount to prevent precipitation of the semiquinoid.

12. The reagent of claim 11, wherein the surfactant is a nonionic or anionic surfactant.

13. A film for detecting the presence of an analyte in a sample, comprising:
a phenazine-containing compound, an enzyme and a film forming agent,
the phenazine-containing compound being of sufficient type to form a semiquinoid as indicator by a reaction involving enzyme, analyte, and the phenazine-containing compound and being in sufficient amount to allow detection of the semiquinoid indicator and thereby detection of the analyte in the sample,
the enzyme being of sufficient type and in sufficient amount to catalyze the indicator-forming reaction involving enzyme, analyte, and phenazine-containing compound, and
the film forming agent being in sufficient amount to form a cohesive film.

14. The film of claim 13, wherein the phenazine-containing compound is in sufficient amount to correlate the concentration of semiquinoid to the concentration of analyte in the sample.

15. The film of claim 13, wherein the analyte is glucose and the amount of phenazine-containing compound is at least about 11 micromoles per gram of dry film.

16. The film of claim 14, wherein the analyte is glucose and the amount of phenazine-containing compound is at least about 18 micromoles per gram of dry film.

17. The film of claim 13 or 14, further comprising: a buffer of sufficient type and in sufficient amount to provide and maintain a proper pH for the reaction involving enzyme, analyte, and phenazine-containing compound.

18. The film of claim 17, further comprising:
a surfactant of sufficient type and in sufficient amount to wet the surface of the film upon addition of the sample.

19. The film of claim 18, wherein the analyte is glucose, the phenazine-containing compound is phenazine ethosulfate, the enzyme is glucose oxidase, the film forming agent is microcrystalline hydroxyethylcellulose, the buffer is malic acid, and the surfactant is polyoxyethylenesorbitan monolaurate.

## Patentansprüche

1. Reagens zum Nachweis der Gegenwart eines Analyten in einer Probe, umfassend:
eine Phenazin enthaltende Verbindung, die von hinlänglicher Art ist, um ein Semichinoid als Indikator durch eine Reaktion unter Beteiligung eines Enzyms, eines Analyten und der Phenazin enthaltenden Verbindung zu bilden, und in ausreichender Menge vorliegt, um den Nachweis des semichinoiden Indikators und damit den Nachweis des Analyten in der Probe zu ermöglichen, und wobei das Enzym von hinlänglicher Art ist und in ausreichender Menge vorliegt, um die indikatorbildende Reaktion unter Beteiligung des Enzyms, des Analyten und der Phenazin enthaltenden Verbindung zu katalysieren.

2. Reagens nach Anspruch 1, des weiteren umfassend Wasser.

3. Reagens nach Anspruch 1, worin die Phenazin enthaltende Verbindung in ausreichender Menge vorliegt, um die Konzentration des Semichinoids mit der Konzentration des Analyten in der Probe zu korrelieren.

4. Reagens nach Anspruch 3, des weiteren umfassend Wasser.

5. Reagens nach Anspruch 2, wobei der Analyt Glucose ist und die Konzentration der Phenazin enthaltenden Verbindung wenigstens etwa einmillimolar ist.

6. Reagens nach Anspruch 4, wobei der Analyt Glucose ist und die Konzentration der Phenazin enthaltenden Verbindung wenigstens etwa 4millimolar ist.

7. Reagens nach Anspruch 4, wobei die Phenazin enthaltende Verbindung ausgewählt ist aus der Gruppe bestehend aus Phenazinmethosulfat, Phenazinethosulfat, N-Ethylmethoxyphenazinethosulfat und 1-Methoxyphenazinmethosulfat.

8. Reagens nach Anspruch 4, wobei die Phenazin enthaltende Verbindung ausgewählt ist aus der Gruppe bestehend aus Phenazinmethosulfat und Phenazinethosulfat.

9. Reagens nach einem der Ansprüche 1 bis 4, des weiteren umfassend einen Puffer hinlänglicher Art und in ausreichender Menge, um für die Reaktion unter Beteiligung des Enzyms, des Analyten und der Phenazin enthaltenden Verbindung den richtigen pH bereitzustellen und aufrechtzuerhalten.

10. Reagens nach Anspruch 8, des weiteren umfassend einen Puffer hinlänglicher Art und in ausreichender Menge, um für die Reaktion unter Beteiligung des Enzyms, des Analyten und der Phenazin enthaltenden Verbindung den richtigen pH bereitzustellen und aufrechtzuerhalten.

11. Reagens nach Anspruch 9, des weiteren umfassend ein Tensid hinlänglicher Art und in ausreichender Menge, um eine Ausfällung des Semichinoids zu verhindern.

12. Reagens nach Anspruch 11, wobei das Tensid ein nichtionisches oder anionisches Tensid ist.

13. Film zum Nachweis der Gegenwart eines Analyten in einer Probe, umfassend:
eine Phenazin enthaltende Verbindung, ein Enzym und ein filmbildendes Mittel,
wobei die Phenazin enthaltende Verbindung von hinlänglicher Art ist, um ein Semichinoid als Indikator durch eine Reaktion unter Beteiligung des Enzyms, des Analyten und der Phenazin enthaltenden Verbindung zu bilden, und in ausreichender Menge vorliegt, um den Nachweis des semichinoiden Indikators und damit den Nachweis des Analyten in der Probe zu ermöglichen,
wobei das Enzym von hinlänglicher Art ist und in ausreichender Menge vorliegt, um die indikatorbildende Reaktion unter Beteiligung des Enzyms, des Analyten und der Phenazin enthaltenden Verbindung zu katalysieren, und wobei das filmbildende Mittel in ausreichender Menge vorliegt, um einen zusammenhängenden Film zu bilden.

14. Film nach Anspruch 13, worin die Phenazin enthaltende Verbindung in ausreichender Menge vorliegt, um die Konzentration des Semichinoids mit der Konzentration des Analyten in der Probe zu korrelieren.

15. Film nach Anspruch 13, wobei der Analyt Glucose ist und die Menge an Phenazin enthaltender Verbindung wenigstens etwa 11 µmol pro Gramm trockenen Films beträgt.

16. Film nach Anspruch 14, wobei der Analyt Glucose ist und die Menge an Phenazin enthaltender Verbindung wenigstens etwa 18 µmol pro Gramm trockenen Films beträgt.

17. Film nach Anspruch 13 oder 14, des weiteren umfassend einen Puffer hinlänglicher Art und in ausreichender Menge, um für die Reaktion unter Beteiligung des Enzyms, des Analyten und der Phenazin enthaltenden Verbindung den richtigen pH bereitzustellen und aufrechtzuerhalten.

18. Film nach Anspruch 17, des weiteren umfassend ein Tensid hinlänglicher Art und in ausreichender Menge, um die Oberfläche des Films nach Zugabe der Probe zu benetzen.

19. Film nach Anspruch 18, wobei der Analyt Glucose ist, die Phenazin enthaltende Verbindung Phenazinethosulfat ist, das Enzym ist Glucoseoxidase ist, das filmbildende Mittel mikrokristalline Hydroxyethylcellulose ist, der Puffer Äpfelsäure ist, und das Tensid Polyoxyethylensorbitanmonolaurat ist.

## Revendications

1. Réactif pour détecter la présence d'un analyte dans un échantillon, comprenant:
un composé contenant une phénazine étant de type suffisant pour former un semi-quinoïde en tant qu'indicateur par réaction impliquant une enzyme, un analyte et le composant contenant une phénazine, et étant en quantité suffisante pour permettre la détection de l'indicateur semi-quinoïde et ainsi la détection de l'analyte dans l'échantillon, et
l'enzyme étant de type suffisant et en une quantité suffisante pour catalyser la réaction formant l'indicateur impliquant une enzyme, un analyte et le composé contenant une phénazine.

2. Réactif selon la revendication 1, comprenant de plus de l'eau.

3. Réactif selon la revendication 1, dans lequel le composé contenant une phénazine est en quantité suffisante pour corréler la concentration en semi-quinoïde à la concentration en analyte dans l'échantillon.

4. Réactif selon la revendication 3, comprenant de plus de l'eau.

5. Réactif selon la revendication 1, dans lequel l'analyte est le glucose et la concentration en composé contenant une phénazine est d'au moins environ 1 millimolaire.

6. Réactif selon la revendication 4, dans lequel l'analyte est le glucose et la concentration en composé contenant une phénazine est d'au moins environ 4 millimolaires.

7. Réactif selon la revendication 4, dans lequel le composé contenant une phénazine est choisi dans un groupe composé du méthosulfate de phénazine, de l'éthosulfate de phénazine, de l'éthosulfate de N-éthylméthoxyphénazine et du méthosulfate de 1-méthoxyphénazine.

8. Réactif selon la revendication 4, dans lequel le composé contenant une phénazine est choisi dans un groupe formé par le méthosulfate de phénazine et l'éthosulfate de phénazine.

9. Réactif selon l'une quelconque des revendications 1 à 4, comprenant de plus: un tampon de type suffisant et en quantité suffisante pour fournir et maintenir un pH correct pour la réaction impliquant l'enzyme, l'analyte et le composé contenant une phénazine.

10. Réactif selon la revendication 8, comprenant de plus: un tampon de type suffisant et en quantité suffisante pour fournir et maintenir un pH correct pour la réaction impliquant l'enzyme, l'analyte et le composé contenant une phénazine.

11. Réactif selon la revendication 9, comprenant de plus: un tensio-actif de type suffisant et en quantité suffisante pour empêcher la précipitation du semi-quinoïde.

12. Réactif selon la revendication 11, dans lequel le tensio-actif est un tensio-actif non ionique ou anionique.

13. Film pour détecter la présence d'un analyte dans un échantillon, comprenant:
un composé contenant une phénazine, une enzyme et un agent filmogène,
le composé contenant une phénazine étant de type suffisant pour former un semi-quinoïde en tant qu'indicateur par une réaction impliquant l'enzyme, l'analyte et le composé contenant une phénazine, et étant en quantité suffisante pour permettre la détection de l'indicateur semi-quinoïde et ainsi la détection de l'analyte dans l'échantillon,
l'enzyme étant de type suffisant et en quantité suffisante pour catalyser la réaction formant un indicateur impliquant l'enzyme, l'analyte et le composé contenant une phénazine, et l'agent filmogène étant en quantité suffisante pour former un film cohésif.

14. Film selon la revendication 13, dans lequel le composé contenant une phénazine est en quantité suffisante pour corréler la concentration du semi-quinoïde à la concentration d'analyte dans l'échantillon.

15. Film selon la revendication 13, dans lequel l'analyte est le glucose et la quantité de composé contenant une phénazine est d'au moins environ 11 micromoles par gramme de film sec.

16. Film selon la revendication 14, dans lequel l'analyte est le glucose et la quantité de composé contenant une phénazine est d'au moins environ 18 micromoles par gramme de film sec.

17. Film selon la revendication 13 ou 14, comprenant de plus: un tampon de type suffisant et en quantité suffisante pour fournir et maintenir un pH correct pour la réaction impliquant l'enzyme, l'analyte et le composé contenant une phénazine.

18. Film selon la revendication 17, comprenant de plus: un tensio-actif de type suffisant et en quantité suffisante pour mouiller la surface du film après addition de l'échantillon.

19. Film selon la revendication 18, dans lequel l'analyte est le glucose, le composé contenant une phénazine est l'éthosulfate de phénazine, l'enzyme est la glucose oxydase, l'agent filmogène est l'hydroxyéthylcellulose microcristalline, le tampon est l'acide malique et le tensio-actif est le monolaurate de polyoxyéthylènesorbitan.
